(19) Europäisches Patentamt
European Patent Office
Office européen des brevets

(11) Publication number: **0 395 099 B1**

(12) **EUROPEAN PATENT SPECIFICATION**

(45) Date of publication of patent specification: **15.03.95** (51) Int. Cl.[6]: **A61L 15/44**

(21) Application number: **90108100.0**

(22) Date of filing: **27.04.90**

# (54) Additives to tampons.

(30) Priority: **27.04.89 US 343965**
**17.04.90 US 508521**

(43) Date of publication of application:
**31.10.90 Bulletin 90/44**

(45) Publication of the grant of the patent:
**15.03.95 Bulletin 95/11**

(84) Designated Contracting States:
**AT BE CH DE ES FR GB IT LI LU NL SE**

(56) References cited:
**EP-A- 0 117 613**
**DE-C- 3 309 530**
**FR-A- 1 307 930**
**FR-M- 522**

(73) Proprietor: **McNEIL-PPC, INC.**
**Van Liew Avenue**
**Milltown**
**New Jersey 08850 (US)**

(72) Inventor: **Brown-Skrobot, Susan, K.**
**5 Agress Court,**
**Hamilton Square,**
**N.J. 08619 (US)**

(74) Representative: **Strehl Schübel-Hopf Groening & Partner**
**Maximilianstrasse 54**
**D-80538 München (DE)**

EP 0 395 099 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Field of the Invention

The present invention relates to the manufacture of absorbent products and especially of absorbent products such as tampons, sanitary napkins, wound dressings and the like which are adapted to absorb body fluids like menstrual fluid, blood and wound exudates. More particularly, the invention relates to the manufacture of catamental tampons which, owing to the presence therein or thereon of certain inhibitory agents, reduce the amount of toxins produced by bacteria coming into contact therewith.

### Background of the Invention

Menstrually occurring toxic shock syndrome (TSS), a severe and sometimes fatal multi-system disease associated with infection or colonization by Staphylococcus aureus (S. aureus) bacteria, has been statistically linked to the use of tampons during menstruation. The disease is believed to be caused by toxic shock syndrome toxin-1 (TSST-1), the toxin produced by the majority of staphylococcal strains isolated from menstrual TSS patients.

Subsequent to the publication of reports associating toxic shock syndrome with the use of tampons, a number of investigators undertook studies designed to evaluate the effect of tampons on growth of S. aureus bacteria as well as the effect of tampons on the production of TSST-1 by those bacteria. Early efforts to elucidate the role of tampons in TSS yielded conflicting data. Schlievert et al. (Obstet. Gynecol., Vol. 64, pp. 666-670, November 1984) studied the effect of tampons on S. aureus to evaluate whether or not tampon components increase growth of S. aureus and production of toxic shock syndrome toxin-1. It was concluded that, under the test conditions of their study, tampon components provide neither nutrients for growth of toxic shock syndrome S. aureus nor factors that induce production of toxic shock syndrome toxin-1 above control levels. After six hour incubation, some commercially available tampons which were tested were inhibitory to bacterial growth and suppressed toxin production. Others suppressed toxin production but did not inhibit cell growth. One tampon inhibited cell growth but increased the amount of toxin produced. On the other hand, Tierno and Hanna (Contraception, Vol. 31, pp 185-194, 1985) reported that in their experiments tampons did stimulate S. aureus to produce TSST-1.

Reiser et al. (J. Clin. Microbiol., Vol. 25, No. 8, pp 1450-1452, August 1987) thereafter reported the results of tests they conducted to determine the effect of four brands of tampons on production of toxic shock syndrome toxin-1. The amount of air available to the tampons which were tested was limited to that contained in sacs (made from cellulose sausage casing with a molecular weight cut-off of less than 10,000) in which the tampons were enclosed during testing. This method was deemed advantageous in that the limited amount of available air was thought to mimic more closely than previously used methods the in vivo condition in the vagina during menstruation with a tampon in place and in that the tampons which were tested were not altered prior to testing. The results of the tests conducted by Reiser et al. indicated that tampons provide increased surface area for the S. aureus bacteria to grow and adequate oxygen for toxin production. No significant inhibition of growth of the staphylococcal bacteria or TSST-1 production by any of the tampons tested was noted.

Robbins et al., publishing in J. Clinical Microbiol., Vol. 25, No. 8, pp. 1446-1449, August 1987 at the same time as Reiser et al., reported the effect of 17 commercially available tampons on TSST-1 toxin production using a disk-membrane-agar (DMA) method, with incubation at 37°C for 19 hours under 5% $CO_2$ in air. Filter membranes overlaying agar medium (with or without blood) in small petri dishes were spread inoculated with a TSST-1 producing strain of S. aureus. Robbins et al. concluded that the main role of tampons in TSS may be that of providing a fibrous surface for heavy colonization and sufficient air for TSST-1 production. In addition, they found evidence of inhibition of TSST-1 production by additives such as the deodorant/surfactant used in a commercially available deodorant tampon and a decrease in TSST-1 production by inhibiting growth of S. aureus as was observed in the case of a different commercially available tampon. It was thought that both inhibition of TSST-1 production and inhibition of S. aureus growth might prove to be important in reducing the risk of TSS.

U.S. Patent No. 4,405,323 to Auerbach discloses a tampon designed to eliminate the hazards of toxic shock syndrome and dysmenorrhea. The tampon has incorporated herein an antibacterial agent which is said to disperse on contact with body fluids and prevent development of the organisms which produce the toxins which cause toxic shock syndrome. Among the antibacterial materials disclosed for use are a povidone-iodine compound, mercury, zinc, penicillin, erythromycin and nitrofurazone.

Patent Cooperation Treaty Publication No. WO 86/05388 (published September 25, 1986) to Kass teaches that the inclusion of a salt of a nontoxic divalent cation in absorptive pads, e.g. catamenial tampons, inhibits additionally include an enzyme to hydrolyze the polymeric acid to its monomeric form.

Canadian Patent No. 1,123,155 to Sipos discloses a catamenial tampon for preventing toxic shock syndrome during menstrual flow. The body of the tampon, which is open at the insertion end and is closed at the withdrawal end, is snugly surrounded in its expanded condition by a fluid proof, thin and flexible membrane. This membrane, which can be made of polyethylene is against the vaginal wall during use of the tampon, is neutral to the vaginal mucosa and is completely impermeable to bacteria, viruses and toxic decomposition products of the menstrual flow.

Canadian Patent No. 1,192,701 to Bardhan discloses a tampon for the absorption of menstrual flow and comprising an inner layer of liquid-absorbent material and an outer layer which surrounds and encloses the inner layer. Menstrual discharge may flow inwardly to the inner layer but the outer layer is impervious to the passage of menstrual fluid outwardly from the inner layer. A plurality of liquid absorbent wicks extending from the inner layer through apertures formed in the outer layer serve as conduits for the flow of menstrual discharge from outside the tampon to the inner layer thereof. The disclosed structure is said to minimize the availability of discharge outside the tampon with a resulting reduction in the likelihood of growth of S. aureus and consequently its production of toxin. This patent also discloses that an antimicrobial compound which is bactericidal or bacteriostatic to S. aureus may be included in the inner layer. The antimicrobial agent may take the form of an antibiotic (such as penicillin, erythromycin, tetracycline or neomycin), a chemotherapeutic agent (such as a sulfonamide) or a disinfectant (such as phenol). The patent states that since the tampon is protected by its outer layer from contact with the vaginal wall, the risk of an allergic or other adverse reaction to the anti-microbial agent is minimized, and since the antimicrobial agent is also protected by the outer layer from contact with menstrual discharge, there is little risk of the destruction of commensal organisms in the vagina or development of resistance to the antimicrobial agent by S. aureus in any menstrual discharge outside the vagina.

S. Notermans et al. (Journal of Food Safety, Vol. 3 (1981), pages 83-88) reported that glyceryl monolaurate, when used in the proportion of 5g per kg. of meat slurry (pH 6.0-6.2) inhibited toxin productions by Clostridium botulinum type A, type B and type E. This article does not mention Staphylococcus aureus nor any toxin(s) produced therefrom nor does it mention absorbent products or toxic shock syndrome.

U.S. Patent No. 4,585,792 to Jacob et al. discloses that L-ascorbic acid when topically applied to the vaginal area of a human female during menses will inactivate toxins known to contribute to Toxic Shock Syndrome. The ascorbic acid compound may be carried by a vaginal tampon. The disclosure of U.S. 4,722,937, is to the same effect.

U.S. Patent No. 4,413,986 to Jacobs discloses a sterile packaged tampon assembly for sterile insertion of a tampon into the vagina and having a guide tube telescoped around an insertion tube and a flexible sheath attached to the inner end of the guide tube and tucked into the inner end of the insertion tube. In use, as the insertion tube is pushed through the guide tube and into the vagina, the flexible sheath is pulled over the inner end of the insertion tube and extends along the exterior thereof. The portion of the insertion tube which is inserted into the vagina is at all times fully sheathed by the flexible sheath.

FR-A-1,307,930 describes a cleaning and deodorizing tampon that cleans the vulva and perineal regions without irritating the skin during application. It is taught therein that this can be achieved by saturating the tampon with a fluid composition having a pH between 5 and 6.5 and consisting mainly of water, a bacteriostatic agent and a surface active agent.

DE-C-33 09 530 describes absorptive materials, which are soft and compatible to the skin without changing the absorptive properties. Furthermore, it is described that the use of glycerides or partial glycerides of fatty acids of coconut oil, namely of C8-C18 acids, preferably together with partial glycerides of castor oil and glycerides of naturally occuring vegetable fatty acids, support healing processes.

## Summary of the Invention

In accordance with the present invention, it has now been discovered that a compound selected from the group consisting of:

a) a monoester of a polyhydric aliphatic alcohol and a fatty acid containing from eight to eighteen carbon atoms and wherein said monoester has at least one hydroxyl group associated with its aliphatic alcohol residue;

b) diesters of a polyhydric aliphatic alcohol and a fatty acid containing from eight to eighteen carbon atoms and wherein said diester has at least one hydroxyl group associated with its aliphatic alcohol

residue; and

c) mixtures of the aforesaid monoesters and diesters

unexpectedly reduces the amount of toxic shock syndrome toxin-1 produced in vitro when said absorbent product is exposed to Staphylococcus aureus bacteria. Therefore, according to the present invention, such a compound can be used in the manufacture of an absorbent product.

The fatty acid portion of the aforementioned monoesters and diesters may be derived from caprylic, capric, lauric, myristic, palmitic and stearic acids, which are saturated fatty acids whose chain lengths, respectively, are $C_8$, $C_{10}$, $C_{12}$, $C_{14}$, $C_{16}$ and $C_{18}$. The fatty acid portion of the aforementioned monoesters and diesters may be derived as well from unsaturated fatty acids having carbon chain lengths also ranging from $C_8$ to $C_{18}$, one example of such unsaturated fatty acids being oleic acid. The preferred fatty acid for use in the practice of the present invention is lauric acid, a saturated fatty acid whose chemical formula is $C_{11}H_{23}COOH$.

As used in this specification and the appended claims, the term "aliphatic" has the meaning usually accorded it in organic chemistry, i.e. "aliphatic" refers to organic compounds characterized by straight - or branched - chain arrangement of the constituent carbon atoms.

As used in this specification and the appended claims, the term "polyhydric" refers to the presence in a chemical compound of at least two hydroxyl (OH) groups. Thus, a polyhydric aliphatic alcohol is one which has at least two hydroxyl groups and in which the carbon backbone is either straight or branched.

Polyhydric alcohols suitable for forming monoesters and/or diesters for use in the practice of the present invention are 1,2-ethanediol; 1,2,3-propanetriol (glycerol); 1,3-propanediol; 1,4-butanediol; 1,2,4-butanetriol and the like. The preferred polyhydric aliphatic alcohol for forming monoesters and diesters for use in the practice of the present invention is 1,2,3-propanetriol (commonly called glycerol) whose formula is $HOCH_2CH(OH)CH_2OH$.

It will be observed that the esters which are useful in the practice of the present invention have at least one hydroxyl group associated with their aliphatic alcohol residue. Thus, it will be understood that the monoester of 1,2-ethanediol and one of the aforementioned fatty acids may be used in the practice of the present invention because said ester, whose general formula is

$$C_nH_{2n+1}-\underset{\underset{O}{\|}}{C}-O-CH_2-CH_2OH$$

has at least one hydroxyl group (i.e. the hydroxyl group at the far right-hand side of the structural formula shown above) in that portion of the ester derived from the aliphatic alcohol 1,2-ethanediol. On the other hand, it will be understood that the diester of 1,2-ethanediol and one of the aforementioned fatty acids cannot be used in the practice of the present invention because said ester, whose general formula is

$$C_nH_{2n+1}-\underset{\underset{O}{\|}}{C}-O-CH_2-CH_2O-\underset{\underset{O}{\|}}{C}-C_nH_{2n+1}$$

does not have at least one hydroxyl group in that portion of the ester derived from the 1,2-ethanediol.

The monoester of glycerol and one of the designated fatty acids may be used in the practice of the present invention because that ester will have two hydroxyl groups associated therewith which are derived from the glycerol. The diester of glycerol and one of the designated fatty acids may also be used because that ester will have one hydroxyl group associated therewith which is derived from the aliphatic alcohol glycerol. Indeed, as will be seen hereinafter, blends of glycerol monolaurate and glycerol dilaurate have been found to be useful in the practice of the present invention. Finally, it will be understood that the triester of glycerol and one of the designated fatty acids cannot be used in the practice, of the present invention because that ester does not have at least one hydroxyl group in that portion thereof which is derived from the aliphatic alcohol, i.e. glycerol.

Preferred esters for use in the practice of the present invention are glyceryl monolaurate, glyceryl dilaurate and mixtures thereof.

In accordance with the invention, the absorbent product contains an amount of the above-described ester which is effective to inhibit the formation of TSS toxin-1 when said product is exposed to S. aureus. For example, effective amounts have been found to be from about 0.1% and higher and, preferably, at least about 0.5% of the specified mono- or diester compound (or mixtures thereof), based on the weight of the absorbent material comprising the absorbent product. As used herein, the term "absorbent material" includes natural or synthetic fibers, films, foams, wood pulp, peat moss, superabsorbent polymers and the like which are capable, either inherently or by virtue of the manner in which they have been assembled, of absorbing liquids such as water, urine, menstrual fluids, blood, wound exudates and the like.

General Procedure For Preparing Tampons of the Invention

During the course of conducting the investigations forming the basis for the present patent application, varying amounts of the aforementioned ester compounds (or mixtures thereof) were added to several different kinds of tampons. These tampons included ones prepared in the laboratories of the assignee of the present patent application as well as commercially available tampons made by several different manufacturers. Tampons of one manufacturer had different weights from those of another manufacturer and, indeed, no two tampons from a given manufacturer had identical weights. The ester compounds to be investigated were dissolved in isopropyl alcohol to form solutions which were then uniformly applied, by pipetting, to the outer surfaces of the various tampons, after which the isopropyl alcohol was evaporated to provide a tampon comprising the ester compound.

In order to ensure that the absorptive capacity of the tampons was not exceeded, it was decided to fix the amount of isopropyl alcohol solution applied to each tampon at four (4) grams in all cases. In view of this desire to hold constant the weight of isopropyl alcohol solution applied to each tampon, it was necessary to vary the concentration of ester compound in the isopropyl alcohol solution in order to vary the level of the selected ester compounds in the tampon. Accordingly, the following general procedure was used to apply a given ester material to a tampon.

Tampons were labeled for identification and weighed to the nearest one-tenth of a gram. The amount of ester required to give the desired concentration in the treated tampon was then calculated. Solutions of the ester in reagent grade isopropyl alcohol were prepared at concentrations such that four (4) grams of the solution contained the amount of ester to be included in the tampon to be tested. In this manner, the amount of ester in a given tampon could be varied while the total weight of the ester/isopropyl alcohol solution used to prepare each individual tampon was held constant at four (4) grams. For example, if the untreated tampon weighed 2.6 grams, then 0.26 gram of ester was required to provide a tampon comprising 10% of the ester based on the weight of the untreated tampon (i.e. 2.6 gram tampon weight x 0.10 = 0.26 gram ester). In this instance, six and one-half (6.5) grams of ester were dissolved in ninety-three and one-half (93.5) grams of reagent grade isopropyl alcohol to give a solution containing six and one-half (6.5%) by weight of ester. Four (4) grams of this solution contained the required 0.26 gram of ester. As another example, if the untreated tampon weighed 2.8 grams, and it was desired that the concentration of ester be one percent (1%) based on the weight of the untreated tampon, a solution containing 0.70 grams of ester and 99.3 grams of isopropyl alcohol was prepared. Four grams of this solution then contained the required 0.028 grams of ester.

As a third example, if the untreated tampon weighed 2.5 grams, and it was desired that the concentration of ester be 0.1% based on the weight of the untreated tampon, a solution containing 0.0625% by weight of ester in isopropyl alcohol was prepared. Four (4) grams of this solution contained the required 0.0025 gram of ester.

All solutions of ester compound in isopropyl alcohol were thoroughly stirred to ensure uniformity. In addition, especially at higher concentrations, the rate of dissolution of the ester could be increased by warming the ingredients to about 60 °C, e.g. in a heated water bath.

Once the tampon had been weighed and the appropriate solution of ester in reagent grade isopropyl alcohol had been prepared in the manner explained above, four (4) grams of the ester/isopropyl alcohol solution (at room temperature) were uniformly applied, by pipetting, to the outer surfaces of the tampon. Tampons were rotated during the application of the ester solution to ensure as uniform an application as possible. The isopropyl alcohol was then evaporated at 70 °C in a hooded drying oven to yield a tampon comprising the desired level of ester compound.

The foregoing procedure was used to prepare all the ester-containing tampons mentioned in the Examples of the present patent application.

Description of the Preferred Embodiments

In Example 1 which follows herein, the invention will be described in detail in connection with a catamenial tampon comprising an absorbent material, a liquid-pervious covering fabric, and an amount of a mixture of glycerol monolaurate and glycerol dilaurate which is effective to inhibit the production of toxic shock syndrome toxin-1 by S. aureus bacteria when said bacteria are brought into contact with the tampon. It will be understood that the principles of the invention apply as well to other absorbent products such as wound dressings, disposable diapers, sanitary napkins and other kinds of tampons, such as those intended for medical, surgical, dental and/or nasal use.

Catamenial tampons comprising rayon fibers as their absorbent material were prepared as follows. The rayon fibers employed were 3-denier, vicose rayon staple fibers having a length of 1-1/8 inches (2.86 cm) and 11-25 crimps per inch (about 4.3 - 9.8 crimps per centimeter). The fibers were 100% vicose rayon, i.e. they were substantially free of all finishes and additives, such as surfactants and the like, commonly used in commercial production.

Using commercially available carding equipment, the above-described rayon fibers were carded into a fibrous web weighing about 520 grains/$yd^2$ (33.6 grams/$meter^2$). The carded web of rayon fibers was gathered into a tubular ribbon having a diameter of about one inch (2.54 cm). This tubular ribbon was thereafter covered in a nonwoven fabric made from heat-fusible fibers and weighing about 0.25 oz/$yd^2$ (7.08 gm/$m^2$). The edges of the heat-fusible nonwoven fabric were overlapped slightly and subsequently heat treated to form a seal. The covered ribbon of rayon fibers was cut into blanks. A white rayon string was pierced and looped through each blank. The blank was then compressed in known fashion to provide a test tampon having a diameter of 0.47 inch (1.2 cm), a length of 1.75 inches (4.44 cm), and a weight of about 2.6 grams. The dangling portion of the withdrawal string was cut from the tampon prior to testing.

A mixture of glycerol monolaurate and glycerol dilaurate, commercially available under the tradename "Lauricidin", was obtained from Lauricidin, Inc. located in East Lansing, Michigan, U.S.A. This mixture, which is hereinafter sometimes referred to as "Lauricidin", was analyzed and found to contain 93 percent by weight of glycerol monolaurate and 3.5 percent by weight of glycerol dilaurate. It is known that Lauricidin has antimicrobial properties and is non-toxic to humans. It has been suggested for use in anti-caries products, insecticides, cosmetic preparations and food compositions.

EXAMPLE 1

Tampons comprising, respectively, 0.1%, 1.0% and 10% by weight of the aforementioned Lauricidin mixture based on the weight of the untreated tampon were prepared using the above-mentioned test tampons. The Lauricidin mixture was applied to the test tampons according to the General Procedure for Preparing Tampons of the Invention described earlier in this application. The Lauricidin containing tampons were prepared in duplicate. The Lauricidin treated tampons were then tested according to the Tampon Sac Method reported by Reiser et al. in the Journal of Clinical Microbiology, Vol. 25, August 1987, pp. 1450-1452, the disclosure of which is hereby incorporated by reference. Staphylococcus aureus strain FRI-1169, obtained in lyophilized form from Dr. Merlin Bergdoll, Food Research Institute, University of Wisconsin, in Madison, Wisconsin, U.S.A., was employed in the tests. A S. aureus suspension was prepared by thoroughly mixing one (1) milligram of the lyophilized S. aureus strain to one (1) milliliter of Brain Heart Infusion (BHI) Broth (obtained from Difco Laboratories, Detroit, Michigan, U.S.A.), transferring said mixture into a test tube containing five (5) milliliters of BHI Broth, thoroughly mixing again, and incubating for twenty-four (24) hours at 37°C prior to use.

100 milliliters of brain heart infusion (BHI) agar (also obtained from Difco Laboratories in Detroit, Michigan, U.S.A.) were put into each of ten 3.8 cm x 20 cm culture tubes. Cellulose sacs were made and sterilized in the manner reported by Reiser et al. The sterile cellulose sacs were inoculated with the aforementioned S. aureus suspension in an amount sufficient to provide at the beginning of the test a concentration therein of 1 x $10^8$ CFU/ml Staphylococcus aureus bacteria.

Each Lauricidin treated tampon to be tested was inserted into a sterile cellulose sac containing the S. aureus bacteria and each sac was then inserted into a culture tube containing the BHI agar. Two controls, each in duplicate, were used. In one control (called the "inoculum control"), an inoculated sac (with no tampon therein) was placed in each of two culture tubes containing BHI agar. In the second control, two untreated tampons (i.e. tampons made in the described manner but with no Lauricidin in the isopropyl alcohol) were placed in cellulose sacs which in turn were placed in culture tubes containing BHI agar. Thus, ten culture tubes were used in this test, four containing the aforementioned controls (two with tampons; two without tampons) and the others containing the aforementioned Lauricidin-treated test tampons in duplicate.

The concentrations of S. aureus strain FRI-1169 and toxic shock syndrome toxin-1 at the outset of the test (0 hours) and after incubation for 24 hours at 37°C are shown in Table 1.

TABLE 1

THE EFFECT OF LAURICIDIN TREATED TAMPONS ON TSST-1 FORMATION BY AND GROWTH OF STAPHYLOCOCCUS AUREUS

| SAMPLE | INITIAL CONCENTRATION OF S. AUREUS CELLS | INITIAL CONCENTRATION OF TSST-1[a)] | FINAL CONCENTRATION OF S. AUREUS CELLS | FINAL CONCENTRATION OF S. AUREUS CELLS[b)] | FINAL CONCENTRATION OF TSST-1[a] |
|---|---|---|---|---|---|
| | (CFU/ml) ($10^8$) | (μg/ml) | (CFU/ml) ($10^8$) | (/ml) | μg/ml |
| No tampon (control) | 1.0 | ND | 100 | 10.0 | 50 |
| Untreated Tampon (control) | 1.0 | ND | 100 | 10.0 | 31 |
| Tampon w/0.1% Lauricidin | 1.0 | ND | 794 | 10.9 | 15 |
| Tampon w/1.0% Lauricidin | 1.0 | ND | 158 | 10.2 | 0.09 |
| Tampon w/10.0% Lauricidin | 1.0 | ND | 316 | 10.5 | 0.08 |

ND = NOT DETECTED. Detection limit of the ELISA method or Reiser et al is 0.5 ng/ml.

a) = As determined by the ELISA method reported by Reiser et al. in Applied and Environmental Microbiology, December 1982, pp. 1349-1355, the disclosure of which is hereby incorporated by reference.

b) = Expressed as log to base 10.

All data above are mean determinations of duplicate samples.

The data in Table 1 show that S. aureus bacteria in the presence of a tampon comprising 0.1% (w/w) Lauricidin produce 51% less toxic shock syndrome toxin-1 (TSST-1) than when exposed to a control tampon containing no Lauricidin, in spite of the fact that there was no reduction in the actual number of viable S. aureus cells in the presence of the Lauricidin-treated tampon. The data further show that S. aureus bacteria in the presence of tampons comprising 1.0% (w/w) and 10.0% (w/w) of Lauricidin produced 99% less TSST-1 than did the same number of S. aureus cells in the presence of a control tampon containing no Lauricidin, again in spite of the fact that there was no reduction in the number of viable S. aureus cells in the presence of Lauricidin-containing tampons. The data show that although the number of viable S. aureus cells does not decrease when the cells are exposed to tampons containing various levels of Lauricidin, the actual amount of TSST-1 produced by those cells is significantly reduced (as in the case of the tampon treated with 0.1% Lauricidin) or substantially eliminated (as in the case of tampons treated with 1.0% and 10% of Lauricidin). In other words, while Lauricidin does not significantly reduce the number of viable S. aureus bacteria cells, it does significantly inhibit the production of toxic shock syndrome toxin-1 by those cells.

Thus, it is believed that in vivo use of Lauricidin treated tampons in menstruating women would be beneficial in that the production of TSST-1 by any S. aureus bacteria normally present in the vagina would be significantly reduced. In addition, in preliminary studies conducted using the predominant microorganism present in the vagina, i.e. Lactobacillus acidophilus, it was found that said microorganism was not adversely affected when brought into contact with an Example 1 tampon comprising 1.0% (w/w) Lauricidin.

Example 2

A second experiment was conducted to evaluate growth of, and TSST-1 production by, S. aureus cells in the presence of commercially available tampons treated with varying amounts of Lauricidin. Tampax* brand menstrual tampons (regular size, Lot No. 8L015Z) which had been purchased on the open market were employed in the experiment of this Example 2. These tampons were manufactured by Tambrands, Inc., Lake Success, New York, U.S.A. The Tampax* tampons comprised a cotton absorbent core, a rayon fabric covering, and a withdrawal string. The tampon withdrawal strings were cut from the tampons prior to testing. Tampons comprising 0.1%, 1.0% and 10% Lauricidin based on the weight of the untreated tampons were prepared in duplicate according to the aforementioned General Procedure. Two Tampax* tampons (with strings cut off), treated with isopropyl alcohol containing no Lauricidin and thereafter dried, were used as a control. The dried, Lauricidin-treated tampons and the control tampons, were then tested according to the procedure and conditions reported in Example 1. The test results are reported in Table 2.

TABLE 2

THE EFFECT OF LAURICIDIN TREATED TAMPAX TAMPONS (LOT NO. 8L015Z) ON TSST-1 FORMATION BY STAPHYLOCOCCUS AUREUS (FRI-1169)

| SAMPLE | FINAL CONCENTRATION OF S. AUREUS CELLS | FINAL CONCENTRATION OF S. AUREUS CELLS[b)] | FINAL CONCENTRATION OF TSST-1[a)] |
|---|---|---|---|
| | (CFU/ml) ($10^8$) | (/ml) | (µg/ml) |
| 0% Lauricidin Tampon (control) | 5,248 | 11.72 | 27.50 |
| 0.1% Lauricidin On Tampon | 199 | 10.30 | 3.70 |
| 1.0% Lauricidin On Tampon | 251 | 10.40 | 1.05 |
| 10.0% Lauricidin On Tampon | 2.13 | 8.33 | 0.19 |

a) = As determined by ELISA method (Reiser et al.).

b) = Expressed as log to base 10.

All sample determinations were made after 24 hrs. incubation at 37 °C.

All data above are mean determinations of duplicate samples.

The data in Table 2 show that S. aureus bacteria in the presence of Tampax* brand tampons treated with varying amounts of Lauricidin produce less TSST-1 than when exposed to the control tampon containing no Lauricidin, the extent of the reduction in toxin production being related to the amount of Lauricidin in the tampons. Tampax* tampons treated with 0.1% by weight of Lauricidin resulted in an 86% reduction in TSST-1 produced compared to the control, while the Tampax* tampons treated with 1% and 10% by weight of Lauricidin resulted in, respectively, a 96% and 99% reduction. The results with the Tampax* brand tampons also show a reduction in the total number of S. aureus cells; this effect is dependent upon the concentration of Lauricidin in the tampon. At the end of the 24 hour incubation period, the log concentration of S. aureus cells in the presence of the control tampon was 11.72; the log concentration of S. aureus cells in the presence of the tampon containing 0.1% Lauricidin was 10.30 (12% less); the log concentration of S. aureus cells in the presence of the tampon containing 1.0% Lauricidin was 10.40 (11% less); and the log concentration of S. aureus cells in the presence of the tampon containing 10% Lauricidin was 8.33 (29% less).

Example 3

A third experiment was conducted to evaluate growth of, and TSST-1 production by, S. aureus cells in the presence of commercially available tampons treated with varying amounts of Lauricidin. Playtex* brand menstrual tampons (regular size, Lot No. 3496P) which had been purchased on the open market were employed in the experiment of this Example 3. These tampons were manufactured by International Playtex Inc., Dover, Delaware, U.S.A. The Playtex* tampons were made of all rayon fiber and had a withdrawal string but no cover fabric. The tampon withdrawal strings were cut from the tampons prior to testing. Treated tampons comprising 0.1%, 1.0% and 10% Lauricidin based on the weight of the untreated tampon were prepared in duplicate according to the aforementioned General Procedure. Two Playtex* tampons (with strings cut off) without any Lauricidin treatment were used as a control. The dried Lauricidin-treated tampons and the untreated controls were then tested according to the procedure and conditions described in Example 1. The test results are reported in Table 3.

TABLE 3

| THE EFFECT OF LAURICIDIN TREATED PLAYTEX TAMPONS (LOT NO. 3496P) ON TSST-1 FORMATION BY STAPHYLOCOCCUS AUREUS (FRI-1169) | | | |
|---|---|---|---|
| SAMPLE | FINAL CONCENTRATION OF S. AUREUS CELLS | FINAL CONCENTRATION OF S. AUREUS CELLS[b)] | FINAL CONCENTRATION OF TSST-1[a)] |
| | (CFU/ml) | (/ml) | (μg/ml) |
| 0% Lauricidin Tampon (control) | 3,388 | 11.53 | 10.86 |
| 0.1% Lauricidin On Tampon | 213 | 10.33 | 2.69 |
| 1.0% Lauricidin On Tampon | 131 | 10.12 | 0.38 |
| 10.0% Lauricidin On Tampon | 3.23 | 8.51 | 0.29 |

a) = As determined by ELISA method (Reiser et al.).
b) = Expressed as log to base 10.
All sample determinations were made after 24 hrs. incubation at 37 °C.
All data above are mean determinations of duplicate samples.

The data reported in Table 3 show that the amount of TSST-1 produced by S. aureus bacteria in the presence of Playtex* tampons treated with 0.1%, 1.0% and 10.0% Lauricidin was reduced 75%, 96% and 97%, respectively, when compared to the amount of TSST-1 produced in the presence of a control tampon containing no Lauricidin. On the other hand, compared to control values, the Playtex* tampons treated with 0.1%, 1.0% and 10.0% of Lauricidin resulted in 10%, 12% and 26% fewer S. aureus cells at the end of the 24 hour incubation period.

Example 4

A fourth experiment was conducted to evaluate growth of, and TSST-1 production by S. aureus cells in the presence of another brand of menstrual tampons treated with varying amounts of Lauricidin. Rely* brand menstrual tampons (regular size, Lot No. 2060LC01A) which had been purchased prior to September 1980 on the open market were employed in the experiment of this Example 4. These tampons were manufactured by Procter Gamble, Cincinnati, Ohio, USA. The Rely* tampons comprised carboxymethyl cellulose dispersed in a polyester foam which was wrapped in a nonwoven fabric made of spunbonded polyester fibers. They had the usual withdrawal strings. The tampon withdrawal strings were cut from the tampons prior to testing. Treated tampons comprising 0.1%, 1.0% and 10% Lauricidin based on the weight of the untreated tampon were prepared in duplicate according to the aforementioned General Procedure. Two Rely* tampons (with their withdrawal strings cut off) without any Lauricidin treatment were used as a control. The dried Lauricidin-treated tampons and the untreated control tampons were then tested according to the procedure and conditions described in Example 1. The test results are reported in Table 4.

TABLE 4

| THE EFFECT OF LAURICIDIN TREATED RELY TAMPONS (LOT NO. 2060LC01A) ON TSST-1 FORMATION BY STAPHYLOCOCCUS AUREUS (FRI-1169) | | | |
|---|---|---|---|
| SAMPLE | FINAL CONCENTRATION OF S. AUREUS CELLS | FINAL CONCENTRATION OF S. AUREUS CELLS[b)] | FINAL CONCENTRATION OF TSST-1[a)] |
| | (CFU/ml) ($10^8$) | (/ml) | ($\mu$g/ml) |
| 0% Lauricidin Tampon (control) | 12,303 | 12.09 | 64.32 |
| 0.1% Lauricidin On Tampon | 2,818 | 11.45 | 6.92 |
| 1.0% Lauricidin On Tampon | 1,995 | 11.30 | 1.54 |
| 10.0% Lauricidin On Tampon | 1,096 | 11.04 | 0.09 |

a) = As determined by ELISA method (Reiser et al.).
b) = Expressed as log to base 10.
All sample determinations were made after 24 hrs. incubation at 37 °C.
All data above are mean determinations of duplicate samples.

The data reported in Table 4 show that the amount of TSST-1 produced by S. aureus bacteria in the presence of the Rely* tampons treated with 0.1%, 1.0% and 10.0% Lauricidin was reduced by 89%, 97% and 99%, respectively, when compared to the amount of TSST-1 produced in the presence of a control tampon containing no Lauricidin. Whereas, at the end of the 24 hour incubation period, the total S. aureus cell concentration (expressed as log to the base 10) in the presence of the control tampon was 12.09, the total S. aureus cell concentration (expressed as log to the base 10) in the presence of the Rely* tampon treated with 0.1%, 1.0% and 10% Lauricidin was, respectively, 11.45 (5.3% less), 11.20 (7.4% less) and 11.04 (8.6% less).

Example 5

A fifth experiment was conducted to evaluate growth of, and TSST-1 production by S. aureus cells in the presence of commercially available tampons treated with varying amounts of Lauricidin. O.b.* brand menstrual tampons (regular size, Lot No. 0694T) which had been purchased on the open market were employed in the experiment of this Example 5. These tampons were distributed by Personal Products Company, Milltown, NJ, U.S.A. The o.b.* tampons comprise a blend of rayon and cotton. They included a withdrawal string but did not have an outer cover sheet. The tampon withdrawal strings were cut from the tampons prior to testing. Treated tampons comprising 0.1%, 1.0% and 10% Lauricidin based on the weight of the untreated tampon were prepared in duplicate according to the General Procedure described earlier herein. Two o.b.* tampons (with strings cut off) without any Lauricidin treatment were used as a control. The

dried Lauricidin-treated tampons and the untreated control tampons were then tested according to the procedure and conditions described in Example 1. The test results are reported in Table 5.

TABLE 5

| THE EFFECT OF LAURICIDIN TREATED O.b.* TAMPONS (LOT NO. 0694T) ON TSST-1 FORMATION BY STAPHYLOCOCCUS AUREUS (FRI-1169) | | | |
|---|---|---|---|
| SAMPLE | FINAL CONCENTRATION OF S. AUREUS CELLS | FINAL CONCENTRATION OF S. AUREUS CELLS[b)] | FINAL CONCENTRATION OF TSST-1[a)] |
| | (CFU/ml) ($10^8$) | (/ml) | (μg/ml) |
| 0% Lauricidin Tampon (control) | 3,388 | 11.53 | 13.46 |
| 0.1% Lauricidin On Tampon | 158 | 10.20 | 3.26 |
| 1.0% Lauricidin On Tampon | 316 | 10.50 | 0.28 |
| 10.0% Lauricidin On Tampon | 95 | 9.98 | 0.19 |

a) = As determined by ELISA method (Reiser et al.).
b) = Expressed as log to base 10.
All sample determinations were made after 24 hrs. incubation at 37° C.
All data above are mean determinations of duplicate samples.

The data presented in Table 5 show that the amount of TSST-1 produced by S. aureus bacteria in the presence of o.b.* tampons comprising 0.1%, 1.0% and 10% by weight Lauricidin was reduced by 75%, 98% and 98%, respectively, when compared to the amount of TSST-1 produced in the presence of a control o.b.* tampon containing no Lauricidin. The total S. aureus cell concentration (expressed as log to the base 10) in the presence of the control tampon was 11.53. The total S. aureus concentration (expressed as log to the base 10) in the presence of the o.b.* tampon treated with 0.1%, 1.0% and 10% by weight of the Lauricidin was, respectively, 10.20 (11% less), 10.50 (8.9% less), and 9.98 (13% less).

Example 6

A sixth experiment was conducted to evaluate growth of, and TSST-1 production by S. aureus cells in the presence of commercially available tampons treated with varying amounts of Lauricidin. Kotex* Security* brand menstrual tampons (regular size, Lot No. 5C0907C) which had been purchased on the open market were employed in the experiment of this Example 6. These tampons were marketed by Kimberly-Clark Corporation, Neenah, Wisconsin, U.S.A. They comprised a blend of 60% cotton and 40% rayon, had the usual withdrawal string, and were covered with a nonwoven fabric made of polypropylene fibers. The tampon withdrawal strings were cut from the tampons prior to testing. Treated tampons comprising 0.1%, 1.0% and 10% Lauricidin based on the weight of the untreated tampon were prepared in accordance with the above-described General Procedure. Two Kotex* Security* tampons (with strings cut off) without any Lauricidin treatment were used as controls. The dried Lauricidin-treated tampons and the untreated control tampons were then tested according to the procedure and conditions described in Example 1. The test results are reported in Table 6.

TABLE 6

| THE EFFECT OF LAURICIDIN TREATED KOTEX SECURITY TAMPONS, LOT NO. 5C0907C ON TSST-1 FORMATION BY STAPHYLOCOCCUS AUREUS (FRI-1169) | | | |
|---|---|---|---|
| SAMPLE | FINAL CONCENTRATION OF S. AUREUS CELLS | FINAL CONCENTRATION OF S. AUREUS CELLS[b)] | FINAL CONCENTRATION OF TSST-1[a)] |
| | (CFU/ml) ($10^8$) | (/ml) | (µg/ml) |
| 0% Lauricidin Tampon (control) | 1,698 | 11.23 | 10.19 |
| 0.1% Lauricidin On Tampon | 194 | 10.29 | 4.90 |
| 1.0% Lauricidin On Tampon | 426 | 10.63 | 0.09 |
| 10.0% Lauricidin On Tampon | 426 | 10.63 | 0.05 |

a) = As determined by ELISA method (Reiser et al.).
b) = Expressed as log to base 10.
All sample determinations were made after 24 hrs. incubation at 37 ° C.
All data above are mean determinations of duplicate samples.

The data presented in Table 6 show that the amount of TSST-1 produced by S. aureus bacteria in the presence of Kotex* tampons comprising 0.1%, 1.0% and 10% by weight of Lauricidin was reduced by 52%, 99% and 99%, respectively, when compared to the amount of TSST-1 produced under the same experimental conditions in the presence of a control Kotex* tampon containing no Lauricidin. The total concentration of S. aureus cells (expressed as log to the base 10) in the presence of the Kotex* tampons containing 0.1%, 1.0% and 10% by weight of Lauricidin was, respectively, 10.29 (8.4% less), 10.63 (5.3% less) and 10.63 (5.3%less).

As can be seen from the preceding Examples 1-6, a variety of tampons, one of which was made by the inventors (Example 1), others of which were commercially available (Examples 2, 3, 5 and 6) and one of which had been commercially available but was subsequently withdrawn from commercial distribution (Example 4), have been treated with varying levels of Lauricidin, a commercially available mixture comprising 93% by weight glycerol monolaurate and 3.5% by weight glycerol dilaurate. The data reported in Tables 1-6 show that, depending on the levels of Lauricidin in the tampons, S. aureus bacteria produce significantly less TSST-1 or, in other words, are inhibited from producing significant amounts of TSST-1 when compared to the amounts of TSST-1 produced, under the same experimental conditions, by S. aureus bacteria in the presence of control tampons containing no Lauricidin.

EXAMPLE 7

Test tampons of the kind used in Example I were used in this Example 7. Test tampons comprising 0.1%, 0.5%, and 1.0% Lauricidin based on the weight of the untreated test tampons were prepared according to the General Procedure described earlier herein and were tested according to the Tampon Sac Method described in Example 1. In this Example 7, however, the tampon sacs were inoculated with different strains of S. aureus bacteria prior to the insertion therein of the Lauricidin treated tampons. The respective strains of S. aureus tested are identified in Table 7. The concentration of S. aureus at the outset of the experiment was $1x10^8$ CFU/ml. TSST-1 producing S. aureus strain FRI-1169 utilized in this Example 7 was obtained from Merlin Bergdoll, Ph.D., Food Research Institute, University of Wisconsin, Madison, Wisconsin U.S.A. TSST-1 producing S. aureus strain designated 1169W was obtained from Fred Quimby, V.M.D., Ph.D., Cornell Medical School, New York, New York, U.S.A. A third S. aureus strain (specifically a substrain of FRI-1169) was isolated from the parent strain and designated TSS Isolate. This TSS Isolate can be obtained in a lyophilized state from S. K. Brown-Skrobot, Ph.D., Personal Products Company, Milltown, New Jersey, U.S.A. A fourth TSST-1 producing S. aureus strain designated Mn8, was obtained from Patrick Schlievert, Ph.D., University of Minnesota, Minneapolis-St. Paul, Minnesota, U.S.A. A fifth TSST-1 producing S. aureus strain designated 1187 was obtained from Keith T. Holland, Ph.D., University of Leeds, Leeds, England. All of the S. aureus strains tested in this example can be obtained from the aforementioned

individuals.

Suspensions of the various strains were prepared as described in Example 1 and used to inoculate the sacs prior to insertion of the tampons which were then tested according to the Tampon Sac Method described in Example 1. Duplicate test tampons without any Lauricidin were used as controls. The test results are reported in Table 7.

The results shown in Table 7 show a reduction in TSST-1 formation with increasing concentration of Lauricidin. This was noted in all five strains which were tested. It was concluded from the test results that the beneficial effects of Lauricidin observed in Examples 1-6 were not specific to any particular TSST-1 producing S. aureus strain.

TABLE 7

THE EFFECT OF LAURICIDIN TREATED TAMPONS ON GROWTH OF AND TSST-1 PRODUCTION BY VARIOUS STRAINS OF S. AUREUS

| TSST-1 PRODUCING STRAIN | CONCENTRATION OF LAURICIDIN[a)] (%) | TOTAL AMOUNT TSST-1 PRODUCED[b)] (µg) |
|---|---|---|
| 1169W (Quimby Strain) | NONE | 56.54 |
| | 0.1 | 17.54 |
| | 0.5 | 0.35 |
| | 1.0 | 0.07 |
| FRI-1169 (Bergdoll Strain) | NONE | 48.75 |
| | 0.1 | 5.06 |
| | 0.5 | 0.04 |
| | 1.0 | 0.03 |
| TSS Isolate (Substrain of FRI-1169) | NONE | 53.04 |
| | 0.1 | 5.25 |
| | 0.5 | 1.27 |
| | 1.0 | 0.48 |
| Mn8 (Schlievert Strain) | NONE | 66.30 |
| | 0.1 | 0.66 |
| | 0.5 | 0.12 |
| | 1.0 | 0.05 |
| 1187 (Holland Strain) | NONE | 46.80 |
| | 0.1 | 6.63 |
| | 0.5 | 0.92 |
| | 1.0 | 0.58 |

a) = based on weight of untreated tampon.

b) = Total TSST-1 per tampon after 24 hr. incubation at 37°C.

All samples were tested for total TSST-1 produced using the ELISA method (Reiser et al.).

EXAMPLE 8

In this Example 8, tampons comprising various fatty acid esters were tested to determine their effect on growth of and TSST-1 formation by S. aureus bacteria (FRI-1169). Test tampons of the kind used in Example 1 were used for this Example 8. All of the test tampons weighed 2.6 grams. 0.65 gram of each fatty acid ester to be tested was dissolved in 99.35 grams of reagent grade or ester mixture isopropyl alcohol. Four (4) grams of each fatty acid ester solution were applied to the outer surfaces of each of two test tampons to provide treated tampons comprising 1% by weight of the ester or ester mixture based on the weight of the untreated test tampon. The alcohol was removed by evaporation at 70 °C, after which the treated tampons were tested according to the Tampon Sac Method described in Example 1. Following is a list of the fatty acid esters which were evaluated:

Tampon No. 1 - A mixture of glyceryl monocaprylate and glyceryl caprate. Caprylic acid is a saturated fatty acid containing 8 carbon atoms. Capric acid is a saturated fatty acid containing 10 carbon atoms. The mixture contained about 38.3% by weight of the caprylate ester, about 36.9% by weight of the caprate ester and about 0.6% free glycerine. The remainder of this mixture contained minor amounts of di- and triesters of the two fatty acids.

Tampon No. 2 - Glyceryl monolaurate of 90-95% purity and containing 0.2% free glycerine and minor amounts of the di- and triesters. Lauric acid is a saturated fatty acid containing 12 carbon atoms.

Tampon No. 3 - Glyceryl monomyristate of 90-95% purity and containing about 0.2% free glycerine and minor amounts of the di- and triesters. Myristic acid is a saturated fatty acid containing 14 carbon atoms.

Tampon No. 4 - Glyceryl monopalmitate of 90-95% purity and containing 0.2% free glycerine and minor amounts of the di- and triesters. Palmitic acid is a saturated fatty acid containing 16 carbon atoms.

Tampon No. 5 - Glyceryl monostearate of 90-95% purity and containing 0.2% free glycerine and minor amounts of the di- and triesters. Stearic acid is a saturated fatty acid containing 18 carbon atoms.

Tampon No. 6 - Glyceryl monooleate of 90-95% purity and containing 0.2% free glycerine and minor amounts of the di- and triesters. Oleic acid is an unsaturated fatty acid containing 18 carbon atoms and one double bond.

In this Example 8, two untreated tampons were used as controls.

The results of the tests are shown in Table 8. The data show that there was a marked reduction in the amount of TSST-1 produced by S. aureus strain FRI-1169 in the presence of the tampons treated with the various fatty acid esters when compared to the amount of TSST-1 produced in the presence of the untreated control tampons. The reduction in the amount of TSST-1 produced ranged from about 90% to 99%, except in the case of the tampon containing glyceryl monostearate. The 60% reduction in TSST-1 production observed in the case of the tampon containing glyceryl monostearate, though not as high as that obtained with the tampons containing other esters, was nevertheless quite substantial and is regarded as significant. No corresponding pattern of reduction in the number of viable S. aureus cells was observed. It should be noted, however, that at the end of the 24 hour incubation period, there were fewer viable S. aureus cells on the treated tampons than on the tampons having no ester treatment.

TABLE 8

| IMPACT OF VARIOUS GLYCERYL ESTER COMPOUNDS ON GROWTH OF AND TSST-1 FORMATION BY STAPHYLOCOCCUS AUREUS (FRI-1169) | | | | |
|---|---|---|---|---|
| SAMPLE | FINAL CONCENTRATION OF S. AUREUS CELLS | FINAL CONCENTRATION OF S. AUREUS CELLS[b)] | TOTAL AMOUNT TSST-1 PRODUCED[a)] | REDUCTION IN TSST-1 FORMATION |
| | (CFU/ml) | (/ml) | (μg) | (%) |
| Control Tampon | $4.20 \times 10^9$ | 9.62 | 17.15 | - |
| Tampon No. 1 | $5.80 \times 10^6$ | 6.76 | 0.18 | 98.9 |
| Tampon No. 2 | $6.56 \times 10^6$ | 6.81 | 0.14 | 99.1 |
| Tampon No. 3 | $5.04 \times 10^8$ | 8.70 | 0.58 | 96.6 |
| Tampon No. 4 | $1.44 \times 10^9$ | 9.15 | 1.75 | 89.7 |
| Tampon No. 5 | $6.08 \times 10^8$ | 8.78 | 6.43 | 62.5 |
| Tampon No. 6 | $6.86 \times 10^8$ | 8.83 | 1.01 | 94.1 |

a) As determined by ELISA method (Reiser et al.)
b) Log to the base 10.
All determinations were made after 24 hours at 37 °C.
All data above are mean determinations of duplicate samples.

EXAMPLE 9

The mixture of glyceryl monolaurate and glyceryl dilaurate used in the experiments reported in Examples 1-7 hereof was obtained from Lauricidin, Inc. under the tradename of Lauricidin. As indicated earlier herein, this mixture was analyzed and found to contain 93% by weight of glyceryl monolaurate and 3.5% by weight of glyceryl dilaurate. Mixtures of glyceryl esters of lauric acid were obtained from two other sources. One such mixture was obtained from Stepan Chemical Company, Maywood, New Jersey U.S.A. under the tradename Kessco. This mixture was analyzed and found to contain 50% by weight of glyceryl monolaurate, and 37% by weight of glyceryl dilaurate. Another such mixture was obtained from Henkel Corporation under the name Monomuls 90-L12 and found to contain 96% by weight of glyceryl monolaurate. No glyceryl dilaurate was detected. Using the aforementioned General Procedure and the same test tampons as those used in Example 1, the following tampons were prepared in duplicate:

- Tampons comprising, respectively, 0.1%, 0.5% and 1.0% of Lauricidin based on the weight of the untreated test tampons;
- Tampons comprising, respectively, 0.1%, 0.5% and 1.0% of the Kessco ester mixture based on the weight of the untreated test tampons; and
- Tampons comprising, respectively, 0.1%, 0.5% and 1.0% of Monomuls 90-L12 mixture based on the weight of the untreated test tampon.

Tampons treated with isopropyl alcohol without any ester therein were employed as controls. All samples were prepared and tested in duplicate according to the Tampon Sac Method described earlier herein. Test results are set forth in Table 9.

TABLE 9

| IMPACT OF VARIOUS GLYCEROL MONOLAURATES ON GROWTH OF AND TSST-1 FORMATION BY STAPHYLOCOCCUS AUREUS (FRI-1169) | | | | | |
|---|---|---|---|---|---|
| SAMPLE | % ADD-ON | FINAL CONCENTRATION OF S. AUREUS CELLS | FINAL CONCENTRATION OF S. AUREUS CELLS[b)] | TOTAL AMOUNT TSST-1 PRODUCED[a)] | REDUCTION IN TSST-1 FORMATION |
| | | (CFU/ml) | (/ml) | ($\mu$g) | (%) |
| Control | 0.0 | $8.8 \times 10^7$ | 7.94 | 69.94 | - |
| Kessco Glyceryl Monolaurate | 0.1 | $1.28 \times 10^8$ | 8.10 | 45.24 | 35 |
| | 0.5 | $4.16 \times 10^7$ | 7.62 | 33.60 | 52 |
| | 1.0 | $3.44 \times 10^7$ | 7.53 | 1.40 | 98 |
| Lauricidin* Glyceryl Monolaurate | 0.1 | $1.4 \times 10^7$ | 7.15 | 4.49 | 93 |
| | 0.5 | $1.3 \times 10^7$ | 7.11 | 3.93 | 94 |
| | 1.0 | $3.2 \times 10^7$ | 7.50 | 0.39 | 99 |
| Monomuls 90-L12 | 0.1 | $1.28 \times 10^8$ | 8.10 | 2.41 | 96 |
| | 0.5 | $4.16 \times 10^7$ | 7.62 | 0.36 | 99 |
| | 1.0 | $3.44 \times 10^7$ | 7.53 | 0.16 | 99 |

Kessco* contained 50% by weight of glyceryl monolaurate.
Lauricidin* contained 93% by weight of glyceryl monolaurate.
Monomuls 90-L12 contained 96% by weight of glyceryl monolaurate.
a) As determined by ELISA method (Reiser et al.)
b) Log to the base 10.
All determinations were made after 24 hours at 37 °C.
All data above are mean determinations of duplicate samples.

It can be seen from the test data set forth in Table 9 that, for any given concentration (i.e. add-ons of 0.1%, 0.5% or 1.0%) of ester mixture in the tampon, the final amount of TSST-1 produced under the described test conditions is inversely proportional to the concentration of glyceryl monolaurate in the ester mixture. Thus, for example, where the amount of ester mixture in the test tampons was held constant at the 0.5% add-on level, the final amounts of TSST-1 declined from 33.60 $\mu$g when the ester mixture contained 50% by weight of glyceryl monolaurate (i.e. Kessco*), to 3.93 $\mu$g when the ester mixture contained 93% by weight GML (i.e. Lauricidin*), to 0.36 $\mu$g where the ester mixture contained 96% glyceryl monolaurate (i.e. Monomuls* 90-L12). Similar reductions in the final amounts of TSST-1 produced were observed where the three ester mixtures were used at 0.1% and 1.0% by weight of the tampon. The results set forth in Table 9 suggest that glyceryl monolaurate (which contains two unreacted hydroxyl groups derived from glycerol) is more effective in inhibiting production of TSST-1 than glyceryl dilaurate (which contains a single unreacted hydroxyl group derived from glycerol).

Example 10: In vivo Activity Of Glyceryl Monolaurate-Impregnated Tampons

Test tampons were made as follows. Avtex rayon (100%) #SN2587 three denier was used as the test fiber. The fiber was scoured to remove Tween 20 and either left as unfinished or coated with glycerol monolaurate (Henkel Monomuls L-90)(hereinafter referred to as "GML"). The analytical determination of the monolaurate content of the material was 96.0%, 2.0% of the 1-3 diester and 2.0% unidentified material. The fiber was coated as follows. (Seventy-five pounds) 33.975kg of rayon fiber was loaded into a holding tank and the tank filled with water ((120 gallons) 454.2 l total). Ammonia ($NH_3$) (29.4% v/v) was added to the

water in the holding tank. The system was then heated to (200 ° F) 93 ° C for 30 minutes. The fiber was then washed with hot water ((150 ° F) 65.5 ° C) three times, the wash water checked for any residual foam evidencing the presence of Tween 20. The fiber was then washed with cold water, (60 ° F) 15.5 ° C.

The fiber was transferred to a centrifuge where it was spun while still hot for 5 minutes to remove excess water. The (75 lbs) 33.975 kg. of rayon initially contained (54 lbs.) 24.46 kg of water. The rayon was then opened by hand and placed back into the holding tank. Two restraining plates were placed over the fiber to reduce agitation and minimize foaming. Hot water ((10 - 200 ° F) 12 - 93 ° C) was added followed by four (5-pound) 2.265 kg samples of GML, each dissolved in (five gallons) 18.925 l of (170 ° F) 77 ° C water. The system was pressurized and heated to (190 ° F) 88 ° C and circulated for 30 minutes. After the system was allowed to drain, the fiber was taken to the centrifuge and spun for 5 minutes. At this point, there was (52 lbs.) 27.556 kg of water (70%) remaining. The temperature of the outside fiber was (160 ° F) 71 ° C while the temperature of the inside fibers was (175-180 ° F) 79.5-82 ° C .

The moist fiber was placed in a belt oven, which was heated to about (250-260 ° F) 121-127 ° C. This heat treatment further opened and dried the rayon fiber. The coated and unfinished fiber were then run through a Rando Webber followed by carding in order to form a workable ribbon from which to make tampons.

After the fiber was scoured or coated, dried and carded the rayon ribbon was used in the production of 2.30g tampons. The fiber was compressed bidirectionally and held in a compression puck for five seconds. After compression, the tampons were placed into (0.62") o.d. 1.575 cm applicators. The tampons were wrapped in cellophane and sealed. Control tampons were labelled (y) while GML-coated tampons were designated (x). The tampons were made as follows: Blanks were made by cutting the rayon into sections (2.75") 6.985cm in length by (3.0") 7.62cm in width. Fiber orientation for length was machine-direciton and for width, was cross-direction. The sections were either built up or torn down to obtain blank weights of 2.28g. The rayon section was then hand-rolled and covered. For control blanks, the cover was 0.25 oz. Enka bicomponent fabric ((2.75" x 4.75"), 6.985 x 12.065cm). For GML blanks, the cover was (0.25 oz). 7.088 g Enka bicomponent fabric coated with 2.4% GML solution. The cover was heat sealed to itself using a hand iron. A 8/5 white rayon string, available from Blue Mountain Industries, was cut to (13.0") 33.02 cm lengths. The string was pierced through one end of each blank at a distance of (5/8") 1.588cm from the end on the piercing unit and then looped. Each blank was tested for anchor strength by manually pulling the strig after looping. The blanks were compressed bidirectionally (side-to-side, then end-to-end) and held in the compression puck for 5 seconds. Immediately after compression, the tampons were placed to Reggie three-piece applicator ((0.62") 1.575 cm o.d.). The pull string was not knotted. The tampons were wrapped in white cellophane sleeves and sealed.

The compressed tampons were analyzed for determination of the concentration of GML on the tampon fibers. The average concentration of GML on the tampons tested was 2.38%.

The in vitro impact of the GML tampons compared to that of the unfinished tampons was evaluated using both the Holland Shake Flask Method and the Reiser Tampon Sac Methods for evaluation on TSST-1 formation by S. aureus. The Tampon Sac Method was described before in Examples 1 and 7. The Shake Flask Method is described below. These methods have been described above in Examples 1 and 7. The results of the determination of the impact of the GML (2.38% w/w) coated tampons is set forth in Tables 10 and 11. Table 10 shows that greater than 99.9% reductions in TSST-1 formation were noted when the tampons were evaluated using the Holland shake flask method using the following: two liter triple-baffle flasks were autoclaved containing 500 ml of Difco Brain Heart Infusion Broth. After sterilization, five ml of a 24-hour old culture of S. aureus strain identified as 1187 was added to the flasks. Either 25.0-gram quantities of test material or no material (in the control flasks) were added to the flasks in duplicate. All flasks were incubated at 37 ° C with shaking at 160 rpm for 24 hours, at which time TSST-1 concentration and total S. aureus cell count determinations were made. TSST-1 level was determined using the ELISA test, while total cell counts were made using Standard Plate Count procedures.

Exposure or the GML and unfinished tampons to S. aureus using the Tampon Sac Method can be seen in Table 11. Reductions in TSST-1 formation ranging from 81.1% in media with blood to 95.9% without are demonstrated, while the impact on the total number of S. aureus cells was either none at all in the presence of blood or 9.1% in tubes without blood.

The Holland shake flask method showed higher antimicrobial activity with the shaking and agitating of the system. This in vitro test method was not predictive of the in vivo situation. In contrast, the Tampon Sac Method of Reiser was found to be preferred in predicting the in vivo situation.

The in vivo evaluation of efficacy was performed as follows. Both control and GML tampons were mailed to the Southwest Research Institute in San Antonio, Texas, for evaluation for reduction to TSST-1 formation by S. aureus in the baboon vagina. Twelve female baboons were identified by immobilization with

Ketamine HCl and vaginal examination for gross evidence of infection.

Unfinished control tampons had 5 ml of a S. aureus toxigenic strain grown in Brain Heart Infusion Broth for 24 hours at 37 °C absorbed onto their distal ends (ends distant from the string). Pre-weighed seed tampons were immediately introduced into the baboon vagina, without the use of a speculum, and the pull string cut. Rectal temperature and indirect systolic blood pressure was taken and recorded. Five milliliter blood samples were taken from the cephalic vein and the serum stored at -70 °C until analysis for the presence of anti-TSST-1 antibody and clinical chemistry could be executed.

Seed tampons were maintained intravaginally for twelve hours. After the first twelve hours, the baboon was immobilized with Ketamine HCl and the tampon was removed. The seed tampon was placed in a pre-weighed (4-oz) 113.4 g.plastic cup. Tampon plus cup were weighed and the amount of tampon associated fluid was calculated. The tampon was transferred to a stomacher bag containing 50 ml of sterile saline (0.9% NaCl) and mixed for 60 seconds. The stomacher fluid was then submitted for quantitative determination of total S. aureus cell count and TSST-1 concentration. The total cell count determinations were made using standard plate count method and TSST-1 concentrations were determined using radioimmunoassay (R.I.A.).

All tampons inserted after the seed tampon were treated as previously described. After removal of the seed tampon, all baboons had control (y) tampons inserted intravaginally to allow for additional growth of S. aureus and TSST-1 production within the vaginal cavity. After twelve hours' additional incubation, the animals were divided in two sets of six whereby six baboons were tested with the control tampons and six with GML coated tampons. After the 48-hour exposure period, all animals had tampons inserted supplemented with 5.0 ml of their own blood serum because of diminished menstrual flow. Total viable S. aureus cell count and TSST-1 level determination were made on all tampons. After all tampons were processed and both toxin and cell count determinations were made, four animals were excluded from the study (two control animals and two GML test animals). In these animals, either the organism was not transferred to the vaginal cavity to initiate an infection, or less toxin or cell levels than those known to have been applied to the seed tampon were found. Table 12 sets forth the data of total toxin per milliliter of tampon associated fluid and total toxin on the tampons with the impact on cell count. The data shown is representative of four animals in each test group.

The data set forth in Table 12 demonstrate considerable decreases in toxin formation in the four animals wearing the GML tampons over those wearing the control tampons alone. Figures 1-4 represent the data of the impact of the GML tampons. Although initially the toxin level in the test animals wearing control tampons had higher TSST-1 levels, the GML tampons brought the level of toxin down significantly over that observed in the controls.

The data representing the total toxin produced per $10^6$ cells of S. aureus, thus normalizing the data with respect to individual cells, demonstrates the significant reduction in the tampons containing GML over the control tampons. A trend of increasing toxin in relation to bacterial cells was noted in the control animals after the addition of blood on the 60-hour tampons. The trend noted in the control animals appears to be a direct impact on the cells themselves and the growth curve. This trend was observed in the animals wearing the GML tampons.

The data set forth in Table 13 show a direct comparison of a percentage of the control of toxin on the tampon-associated fluid and total toxin formed in the tampons in comparison with the control tampons.

TABLE 10

| IMPACT OF GML COATED TAMPONS ON TSST-1 FORMATION USING HOLLAND SHAKE FLASK METHOD | | | | | |
|---|---|---|---|---|---|
| SAMPLE | MEDIUM | TOTAL TSST-1 PRODUCED (µg) | REDUCTION (%) | FINAL CONC. OF VIABLE S. AUREUS CELLS (cfu/ml) | TOTAL AMOUNT OF VIABLE S. AUREUS CELLS (cfu) |
| S. aureus | BHI | 81.57 | - | $2.4 \times 10^9$ | $1.20 \times 10^{12}$ |
| Control Tampon | BHI | 1.62 | - | $1.92 \times 10^9$ | $9.60 \times 10^{11}$ |
| GML (2.38%) Tampon | BHI | <0.001 | 99.93 | <10 | $<5.00 \times 10^3$ |
| S. aureus | Blood* | 66.89 | - | $2.0 \times 10^9$ | $1.00 \times 10^{12}$ |
| Control Tampon | Blood | 6.23 | - | $2.40 \times 10^9$ | $1.20 \times 10^{12}$ |
| GML (2.38%) Tampon | Blood | 0.004 | 99.92 | $1.92 \times 10^4$ | $9.60 \times 10^6$ |

* Denotes defribrineated Sheep Blood added to BHI at 1.0% v/v concentration.

TABLE 11

IMPACT OF GML COATED TAMPONS ON TSST-1 FORMATION USING THE REISER TAMPON SAC METHOD

| SAMPLE | MEDIUM | TOTAL TSST-1 PRODUCED (µg) | REDUCTION (%) | CONC. OF VIABLE *S. AUREUS* CELLS (cfu/ml) | TOTAL AMOUNT OF VIABLE *S. AUREUS* CELLS (cfu) |
|---|---|---|---|---|---|
| *S. aureus* | BHI[(a)] | 40.00 | - | $6.0 \times 10^8$ | $4.68 \times 10^9$ |
| Control Tampon | BHI | 65.06 | - | $6.0 \times 10^8$ | $4.68 \times 10^9$ |
| GML Tampon | BHI | 2.63 | 95.9 | $8.0 \times 10^7$ | $6.24 \times 10^8$ |
| *S. aureus* | Blood[(b)] | 54.27 | - | $8.4 \times 10^8$ | $6.55 \times 10^9$ |
| Control Tampon | Blood | 50.55 | - | $1.5 \times 10^7$ | $1.17 \times 10^8$ |
| GML Tampon | Blood | 9.52 | 81.1 | $3.6 \times 10^7$ | $2.80 \times 10^8$ |

(a) Denotes Brain Heart Infusion Agar.
(b) Denotes defribrineated Sheep Blood added to BHI at 1.0% v/v concentration.

TABLE 12

IN VIVO EVALUATION OF THE IMPACT OF GML COATED TAMPONS ON TSST-1 PRODUCTION BY S. AUREUS (1169W).

| TAMPON TYPE | TIME PERIOD (hr.) | CONC. OF TSST-1 IN TAMPON ASSOCIATED FLUID (ng/ml) | TOTAL TAMPON TOXIN (ng) | CONC. OF VIABLE S. AUREUS CELLS* ($x 10^6$cfu/ml) | TOTAL S. AUREUS CELLS ON TAMPON** ($x 10^7$ cfu) | TOTAL TAMPON TOXIN $10^6$ organisms |
|---|---|---|---|---|---|---|
| CONTROL ANIMALS | | | | | | |
| CONTROL | 24 | 987 | 2104 | 38.8 | 11.5 | 18.29 |
| CONTROL | 36 | 534 | 4471 | 20.1 | 12.8 | 34.92 |
| CONTROL | 48 | 1225 | 9435 | 24.3 | 14.3 | 65.97 |
| CONTROL | 60 | 154 | 1015 | 6.17 | 4.0 | 25.37 |
| CONTROL | 72 | 112 | 898 | 3.87 | 2.87 | 31.28 |
| CONTROL | 84 | 72 | 630 | 1.38 | 1.13 | 55.75 |
| TEST ANIMALS | | | | | | |
| CONTROL | 24 | 1136 | 7224 | 55.0 | 10.8 | 66.88 |
| GML | 36 | 583 | 1465 | 38.2 | 9.36 | 15.65 |
| GML | 48 | 355 | 1347 | 28.3 | 9.63 | 13.98 |
| GML | 60 | 62 | 426 | 17.5 | 13.2 | 3.22 |
| GML | 72 | 5.0 | 51 | 3.92 | 3.23 | 1.57 |
| GML | 84 | 0 | 0 | 0.41 | 0.33 | 0 |

* SEED TAMPON FOR CONTROL ANIMALS AVERAGED $2.14 X 10^8$ CFU/ML WHILE TEST ANIMALS HAD $5.86 X 10^7$ CFU/ML

** SEED TAMPON FOR CONTROL ANIMALS AVERAGED $1.98 X 10^9$ CFU WHILE TEST ANIMALS HAD $5.55 X 10^8$ CFU

TABLE 13

| PERCENT OF CONTROL OF TOXIN FORMATION | | |
|---|---|---|
| TAMPON TYPE | PERCENT CONTROL OF TOXIN IN TAMPON ASSOCIATED FLUID (%) | PERCENT CONTROL OF TOTAL TOXIN ON TAMPON FORMED (%) |
| CONTROL ANIMALS | | |
| CONTROL | - | - |
| CONTROL | 199 | 294 |
| CONTROL | 134 | 102 |
| CONTROL | 29 | 40 |
| CONTROL | 56 | 77 |
| CONTROL | 41 | 119 |
| TEST ANIMALS | | |
| CONTROL | - | - |
| GML | 46 | 30.5 |
| GML | 33.5 | 20.25 |
| GML | 6.75 | 5.75 |
| GML | 3.25 | 4.0 |
| GML | 4 | <1.0 |

TABLE 14

| IMPACT OF VARIOUS FATTY ACID ESTER COMPOUNDS ON GROWTH AND TSST-1 FORMATION | | | |
|---|---|---|---|
| Sample | Log of Final Concn. of S. aureus (/ml) | Total Amount TSST-1 Produced (µg) | Reduction in TSST-1 Formation (%) |
| Inoc. 1169 | 11.0 | 15.45 | -- |
| Control Tampon | 9.60 | 8.566 | -- |
| Tampon 1 | | | |
| 0.1% | 9.1 | 7.93 | 7% |
| 0.5% | 7.64 | 1.25 | 85% |
| 1.0% | 8.79 | 0.09 | 99% |
| 10% | 8.37 | 0.05 | 99% |
| Tampon 2 | | | |
| 0.1% | 8.45 | 0.12 | 99% |
| 0.5% | 7.40 | 0.01 | 99% |
| 1.0% | 6.13 | 0.01 | 99% |
| 10% | 7.42 | 0.11 | 99% |

Example 11

In this Example 11, tampons comprising various fatty acid esters were tested to determine their effect on growth of and TSST-1 formation by S. aureus bacteria (FRI-1169). Test tampons of the kind used in Example 1 were used for this Example 11. Test tampons comprising 0.1%, 0.5%, 1.0% and 10% fatty acid ester based on the weight of the untreated test tampons were prepared according to the General Procedure described earlier herein and were tested according to the Tampon Sac Method described in Example 1. Duplicate test tampons without any fatty acid ester were used as controls. The test results are reported in Table 14 above.

The following is a list of the fatty acid esters which were evaluated:

Tampon No. 1- 2-Hydroxy-1-propyl laurate

Tampon No. 2- Diethylene glycol monolaurate

The test results show that there was a marked reduction in the amount of TSST-1 produced by S. aureus strain FRI-1169 in the presence of the tampons treated with the various fatty acid esters when compared to the amount of TSST-1 produced in the presence of the untreated control tampons. The reduction in the amount of TSST-1 produced ranged from about 7% (in the case of 0.1% 2-hydroxy-1-propyl-laurate) to about 99% (in the case of 1.0% 2-hydroxy-1-propyl-laurate and diethyleneglycol monolaurate). No corresponding pattern of reduction in the number of viable S. aureus cells was observed.

## Claims

**1.** Use of a compound selected from the group consisting of:
  a) monoesters of a polyhydric aliphatic alcohol and a fatty acid containing from eight to eighteen carbon atoms and wherein said monoester has at least one hydroxyl group associated with its aliphatic alcohol residue;
  b) diesters of a polyhydric aliphatic alcohol and a fatty acid containing from eight to eighteen carbon atoms and wherein said diester has at least one hydroxyl group associated with its aliphatic alcohol residue; and/or
  c) mixtures of said monoesters and diesters

for the manufacture of an absorbent product inhibiting the production of toxic shock syndrome toxin-1 by Staphylococcus aureus bacteria.

**2.** Use according to Claim 1 wherein said absorbent product comprises absorbent material in which said compound in present in an amount which is at least 0.1 % based on the weight of said absorbent material.

**3.** Use according to anyone of the preceeding claims wherein said compound is present in an amount which is at least 0.5 % based on the weight of said absorbent material.

**4.** Use according to anyone of the preceeding claims wherein said compound is present in an amount which is at least 1 % based on the weight of said absorbent material.

**5.** Use according to anyone of the preceeding claims wherein said fatty acid is lauric acid.

**6.** Use according to anyone of the preceeding claims wherein said polyhydric alcohol is glycerol.

**7.** Use according to anyone of the preceeding claims wherein said compound is glyceryl monolaurate.

**8.** Use according to anyone of the preceeding claims wherein said compound comprises a mixture of glyceryl monolaurate and glyceryl dilaurate.

**9.** Use according to anyone of the preceeding claims wherein said mixture comprises at least 93 % by weight of glyceryl monolaurate.

**10.** Use according to anyone of the preceeding claims wherein said mixture comprises at least 95 % by weight of glyceryl monolaurate.

**11.** Use according to anyone of the preceeding claims wherein said compound comprises glyceryl monocaprylate.

**12.** Use according to anyone of the preceeding claims wherein said compound comprises glyceryl caprate.

**13.** Use according to anyone of the preceeding claims wherein said compound comprises a mixture of glyceryl monocaprylate and glyceryl caprate.

**14.** Use according to anyone of the preceeding claims wherein said compound comprises glyceryl monomyristate.

**15.** Use according to anyone of the preceeding claims wherein said compound comprises glyceryl monopalmitate.

**16.** Use according to anyone of the preceeding claims wherein said compound comprises glyceryl monostearate.

**17.** Use according to anyone of the preceeding claims wherein said compound comprises glyceryl monooleate.

**18.** Use according to anyone of the preceeding claims wherein said absorbent product is a catamenial tampon.

**Patentansprüche**

**1.** Verwendung einer Verbindung, ausgewählt aus der Gruppe bestehend aus:

a) Monoestern eines mehrwertigen aliphatischen Alkohols und einer Fettsäure, die von acht bis achtzehn Kohlenstoffatome enthält, und wobei dieser Monoester mindestens eine Hydroxylgruppe aufweist, die an seinem aliphatischen Alkoholrest sitzt;

b) Diestern eines mehrwertigen aliphatischen Alkohols und einer Fettsäure, die von acht bis achtzehn Kohlenstoffatome enthält, und wobei dieser Diester mindestens eine Hydroxylgruppe aufweist, die an seinem aliphatischen Alkoholrest sitzt; und/oder

c) Gemischen dieser Monoester und Diester

zur Herstellung eines absorbierenden Produkts, das die Bildung des toxischen Schocksyndrom-Toxins-1 durch Staphylococcus aureus-Bakterien inhibiert.

**2.** Verwendung nach Anspruch 1, wobei das absorbierende Produkt absorbierendes Material umfaßt, in dem die Verbindung in einer Menge vorliegt, die mindestens 0,1 % beträgt, bezogen auf das Gewicht des absorbierenden Materials.

**3.** Verwendung nach einem der vorhergehenden Ansprüche, wobei die Verbindung in einer Menge vorliegt, die mindestens 0,5 % beträgt, bezogen auf das Gewicht des absorbierenden Materials.

**4.** Verwendung nach einem der vorhergehenden Ansprüche, wobei die Verbindung in einer Menge vorliegt, die mindestens 1 % beträgt, bezogen auf das Gewicht des absorbierenden Materials.

**5.** Verwendung nach einem der vorhergehenden Ansprüche, wobei die Fettsäure Laurinsäure ist.

**6.** Verwendung nach einem der vorhergehenden Ansprüche, wobei der mehrwertige Alkohol Glycerin ist.

**7.** Verwendung nach einem der vorhergehenden Ansprüche, wobei die Verbindung Glycerinmonolaurat ist.

**8.** Verwendung nach einem der vorhergehenden Ansprüche, wobei die Verbindung ein Gemisch aus Glycerinmonolaurat und Glycerindilaurat umfaßt.

**9.** Verwendung nach einem der vorhergehenden Ansprüche, wobei das Gemisch mindestens 93 Gewichts-% Glycerinmonolaurat umfaßt.

**10.** Verwendung nach einem der vorhergehenden Ansprüche wobei das Gemisch mindestens 95 Gewichts-% Glycerinmonolaurat umfaßt.

**11.** Verwendung nach einem der vorhergehenden Ansprüche, wobei die Verbindung Glycerinmonocaprylat umfaßt.

**12.** Verwendung nach einem der vorhergehenden Ansprüche, wobei die Verbindung Glycerincaprinat umfaßt.

**13.** Verwendung nach einem der vorhergehenden Ansprüche, wobei die Verbindung ein Gemisch aus Glycerinmonocaprylat und Glycerincaprinat umfaßt.

**14.** Verwendung nach einem der vorhergehenden Ansprüche, wobei die Verbindung Glycerinmonomyristat umfaßt.

**15.** Verwendung nach einem der vorhergehenden Ansprüche, wobei die Verbindung Glycerinmonopalmitat umfaßt.

**16.** Verwendung nach einem der vorhergehenden Ansprüche, wobei die Verbindung Glycerinmonostearat umfaßt.

**17.** Verwendung nach einem der vorhergehenden Ansprüche, wobei die Verbindung Glycerinmonooleat umfaßt.

**18.** Verwendung nach einem der vorhergehenden Ansprüche, wobei das absorbierende Produkt ein Monatstampon ist.

**Revendications**

**1.** Utilisation d'un composé choisi dans le groupe constitué par:
a) des monoesters d'un polyol aliphatique et d'un acide gras contenant de huit à dix-huit atomes de carbone, ledit monoester comportant, associé à son résidu aliphatique, au moins un groupe hydroxyle;
b) des diesters d'un polyol aliphatique et d'un acide gras contenant de huit à dix-huit atomes de carbone, ledit diester comportant, associe à son résidu aliphatique, au moins un groupe hydroxyle; et
c) des mélanges desdits monoesters et diesters;
pour la fabrication d'un produit absorbant inhibant la production de la toxine-1 du syndrome du choc toxique par des bactéries Staphylococcus aureus.

**2.** Utilisation selon la revendication 1, dans laquelle ledit produit absorbant comprend un matériau absorbant dans lequel ledit composé est présent dans une proportion qui est d'au moins 0,1% par rapport au poids dudit matériau absorbant.

**3.** Utilisation selon l'une quelconque des revendications précédentes, dans laquelle ledit composé est présent dans une proportion qui est d'au moins 0,5%, par rapport au poids dudit matériau absorbant.

**4.** Utilisation selon l'une quelconque des revendications précédentes, dans laquelle ledit composé est présent dans une proportion qui est d'au moins 1%, par rapport au poids dudit matériau absorbant.

**5.** Utilisation selon l'une quelconque des revendications précédentes, dans laquelle ledit acide gras est l'acide laurique.

**6.** Utilisation selon l'une quelconque des revendications précédentes, dans laquelle ledit polyol est le glycérol.

**7.** Utilisation selon l'une quelconque des revendications précédentes, dans laquelle ledit composé est le monolaurate de glycéryle.

**8.** Utilisation selon l'une quelconque des revendications précédentes, dans laquelle ledit composé comprend un mélange de monolaurate de glycéryle et de dilaurate de glycéryle.

**9.** Utilisation selon l'une quelconque des revendications précédentes, dans laquelle ledit mélange comprend au moins 93% en poids de monolaurate de glycéryle.

**10.** Utilisation selon l'une quelconque des revendications précédentes, dans laquelle ledit mélange comprend au moins 95% en poids de monolaurate de glycéryle.

**11.** Utilisation selon l'une quelconque des revendications précédentes, dans laquelle ledit composé comprend du monocaprylate de glycéryle.

**12.** Utilisation selon l'une quelconque des revendications précédentes, dans laquelle ledit composé comprend du caprate de glycéryle.

**13.** Utilisation selon l'une quelconque des revendications précédentes, dans laquelle ledit composé comprend un mélange de monocaprylate de glycéryle et de caprate de glycéryle.

**14.** Utilisation selon l'une quelconque des revendications précédentes, dans laquelle ledit composé comprend du monomyristate de glycéryle.

**15.** Utilisation selon l'une quelconque des revendications précédentes, dans laquelle ledit composé comprend du monopalmitate de glycéryle.

**16.** Utilisation selon l'une quelconque des revendications précédentes, dans laquelle ledit composé comprend du monostéarate de glycéryle.

**17.** Utilisation selon l'une quelconque des revendications précédentes, dans laquelle ledit composé comprend du monooléate de glycéryle.

**18.** Utilisation selon l'une quelconque des revendications précédentes, dans laquelle ledit produit absorbant est un tampon périodique.

## TSST-1 toxin production

TSST-1 per milliliter of tampon-associated fluid

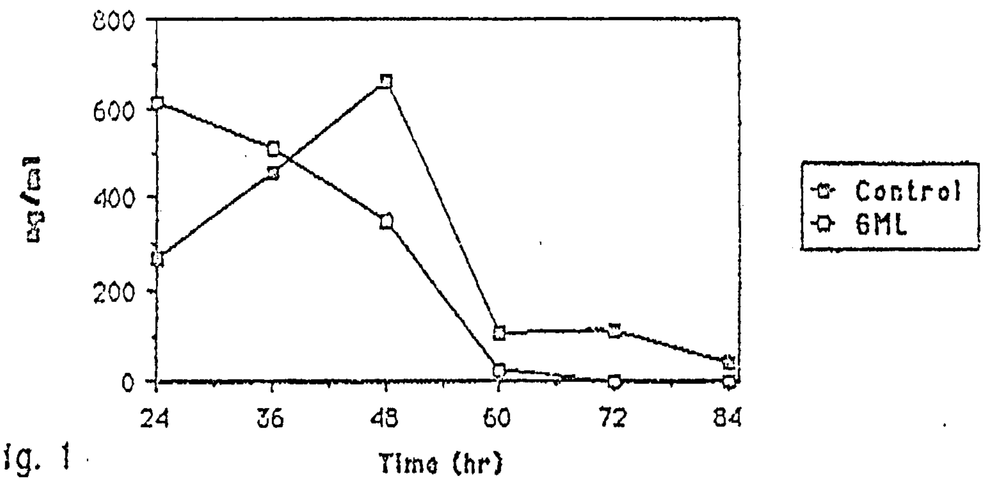


Fig. 1

Total tampon toxin production

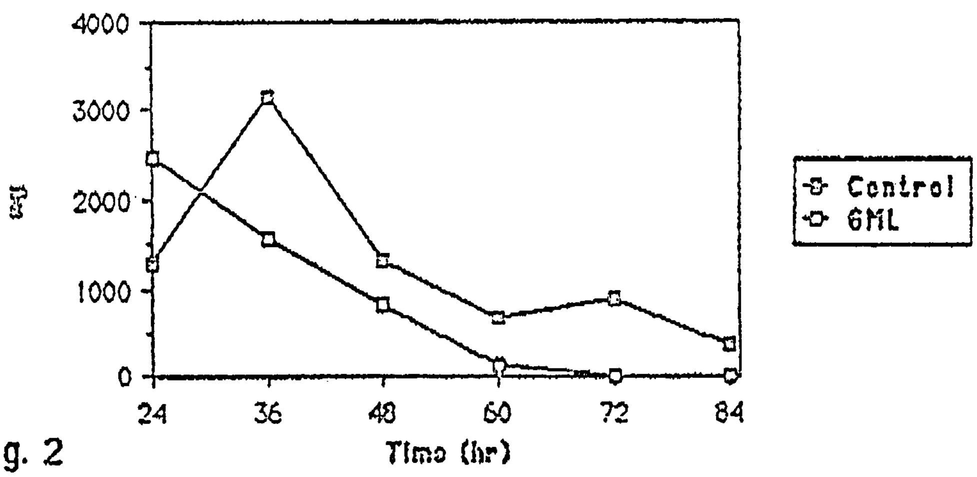


Fig. 2

TSST-1 toxin production as % of control (initial) exposure

TSST-1 (ng) per milliliter of TAF

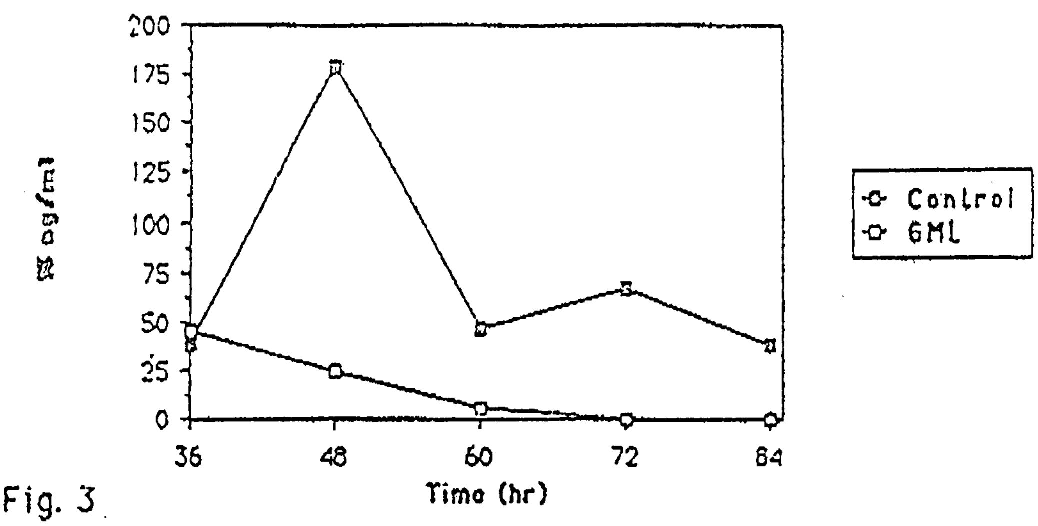


Fig. 3

Total tampon associated TSST-1

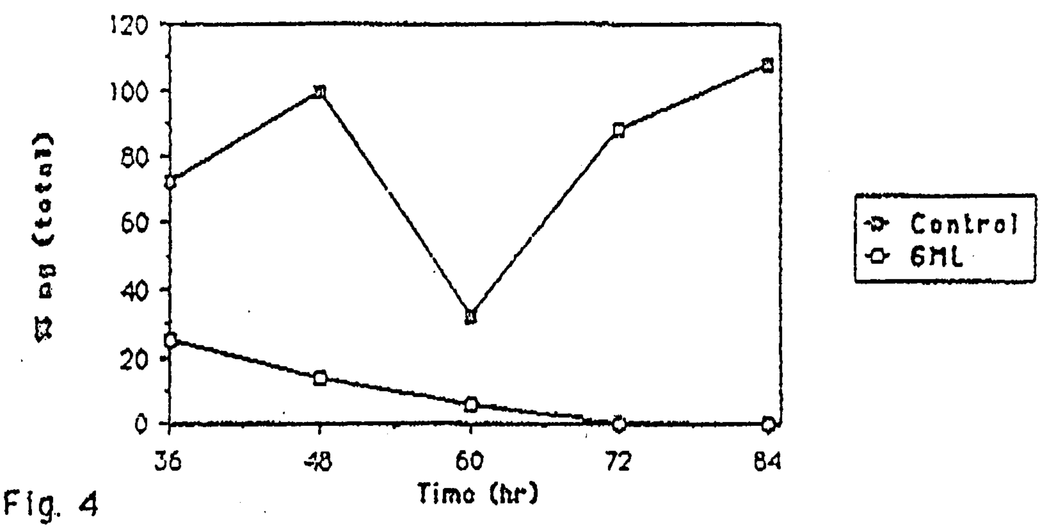


Fig. 4